# EUROPÄISCHE PATENTSCHRIFT

(11) **EP 3 886 733 B1**
(45) Veröffentlichungstag und Bekanntmachung des Hinweises auf die Patenterteilung: **19.10.2022**
(21) Anmeldenummer: 19813759.8
(22) Anmeldetag: 28.11.2019
(51) Int. Cl.: A61B 17/15, A61B 17/00

(54) **FIXIERUNGSSYSTEM UND AUSRICHTUNGSVORRICHTUNG**
FIXING SYSTEM AND ALIGNING DEVICE
SYSTÈME DE FIXATION ET DISPOSITIF D'ORIENTATION

(30) Priorität: 28.11.2018 DE 102018130119
(43) Veröffentlichungstag der Anmeldung: 06.10.2021
(73) Patentinhaber: Aesculap AG, 78532 Tuttlingen (DE)
(72) Erfinder: HIRT, Martin, 78333 Stockach (DE); LINDNER, Stephan, 78573 Wurmlingen (DE)
(74) Vertreter: Winter, Brandl - Partnerschaft mbB
(86) Internationale Anmeldenummer: PCT/EP2019/082964
(87) Internationale Veröffentlichungsnummer: WO 2020/109502

(56) Entgegenhaltungen:
- WO-A1-2013/134595
- GB-A- 2 398 010
- US-A- 5 197 944
- US-A1- 2006 189 998

## Beschreibung

### Technisches Gebiet

Die vorliegende Erfindung betrifft ein Fixierungssystem für eine Ausrichtungsvorrichtung einer tibialen Resektionsführung zur fixierenden Anbindung an einer Körperextremität/einem Körperglied, wie etwa an einem Knöchel, an einem Bein oder an einem Sprunggelenk, mit einer Trägervorrichtung, die einen Anbindungsabschnitt zur Anbindung an einen Einstellstab aufweist. Daneben betrifft die Erfindung eine Ausrichtungsvorrichtung / Einstellführung gemäß dem Oberbegriff des nebengeordneten Anspruchs.

### Hintergrund der Erfindung

Eine präzise Resektion von Knochen eines Patienten, insbesondere der Tibia, hat eine große Bedeutung für einen Erfolg einer Operation zur Implantation einer Gelenksprothese. Die Ebene der Resektion muss hierbei exakt lokalisiert werden, um einerseits ein Maß einer Knochenentnahme möglichst geringzuhalten, wobei andererseits gleichzeitig gewährleistet werden muss, dass auch das gesamte defekte Knochengewebe entfernt wird. Der Abgleich der Ebene in Bezug zu einer anatomischen Achse, insbesondere einer Tibia-Achse, muss bei einer Operation kontinuierlich kontrolliert werden, um die Ausrichtung der Gelenksoberflächen des Gelenks über den gesamten Bereich der Gelenksbewegung zu gewährleisten.

Die exakte Festlegung einer tibialen Resektionsebene in einem Kniegelenk wird üblicherweise unter Verwendung einer Ausrichtungsvorrichtung bzw. einer Einstellführung (für einen Sägeblock) mit einem (säulenförmigen) Einstellstab eingestellt, der entfernt zur Tibia nahe am Fußknöchel befestigt wird. Der Einstellstab erstreckt sich dabei entlang der Tibia (im Wesentlichen) parallel zu der zugehörigen anatomischen Tibia-Achse. Die Resektionsebene kann dann in Bezug auf die Tibia-Achse definiert werden. Ein an der Ausrichtungsvorrichtung befestigter Säge- bzw. Führungsblock (Schneidblock / Führungsvorrichtung) definiert schließlich die Ebene der Resektion. Üblicherweise weist der Führungsblock dafür einen Schlitz auf, durch welchen eine sich hin- und herschwenkende, plane Schneide eines chirurgischen Instruments (Säge) hindurchgeführt wird.

Um die Ausrichtung der Tibia Resektionsebene einzustellen, wird nahe dem Fußknöchel eine Fixierungsklammer angebracht, welche an einem einen zu einem Fuß des Patienten hin weisenden Ende des Einstellstabes angelenkt ist, an dessen anderem Ende (Endabschnitt) der Säge-/Führungsblock fixiert/fixierbar ist.

### Stand der Technik

In dem Stand der Technik werden unterschiedliche Formen einer Ausrichtungsvorrichtung und einer zugehörigen Fixierungsklammer bzw. eines zugehörigen Fixierungssystems offenbart.

Die US 2016/0367291 A1 offenbart beispielsweise eine Einstellführung mit zwei stabförmigen Komponenten, welche an ihren Endabschnitten jeweils ein Kugelgelenk aufweisen und im Wesentlichen symmetrisch zu der Tibia-Achse links und rechts des Fußes bzw. des Beines angeordnet sind. Eine Fixierungsklammer in Form eines U-förmigen Rahmens mit Schrauben, welche in das Fersenbein gebohrt werden, fixiert die beiden stabförmigen Komponenten. Die Einstellführung weist eine Vielzahl an Einstellmöglichkeiten für eine Ausrichtung eines Führungsblockes bzw. einer Führungsvorrichtung auf. Nachteilig ist jedoch, dass für eine Fixierung Knochensubstanz durch das Einschrauben in das Fersenbein unnötigerweise entnommen und gesundes Knochengewebe beschädigt wird. Auch kann die distale Fixierung nachträglich nicht verändert und eingestellt werden.

Die US 6,221,035 B1 offenbart eine (Fixierungs-)Klammer für eine Ausrichtungshilfe, welche bei einer tibialen Resektion verwendet wird. Die Klammer weist zwei federvorgespannte Klammerarme auf, die gegenüber einem Rahmen um jeweils eine Drehachse gedreht werden können. Diese Klammerarme werden in eine geöffnete Position gebracht und nach Anlage an das Schienbein hiernach mittels einer manuellen Auslösevorrichtung freigegeben. Sie umschließen bzw. umgreifen dann aufgrund der Federvorspannung ein Fußgelenk bzw. die Tibia und klemmen diese ein. Die Klammerarme sind dabei in die Schließ-Richtung vorgespannt. Die Federvorspannung bewirkt eine kraftschlüssige Fixierung, was jedoch zum Nachteil hat, dass beim Patienten an den betreffenden Körperstellen aufgrund der Klemmkraft Hämatome entstehen können. Auch kann eine Klemmkraft nicht eingestellt werden.

Die US 2017/0245893 A1 offenbart ebenfalls eine Ausrichtungsvorrichtung sowie eine zugehörige Fixierungsvorrichtung. Hierbei wird in einer Ausführungsform eine Schraube durch das Fersenbein gebohrt, um einen Fixpunkt der Ausrichtungsvorrichtung festzulegen. Auch die CN 205379351 U offenbart eine Einstellführung, bei der Schrauben in Fußknochen zur Fixierung eingeschraubt werden.

Die US 5,197,944 A offenbart ein orthopädisches Instrument und insbesondere eine Knöchel-Klammer bzw. Fixierungsklammer, die es dem Chirurgen ermöglicht, die Knöchel-Klammer als Teil der tibialen Ausrichtungsanordnung mit einer Hand an einem Patienten zu befestigen. Diese Klammer weist insbesondere eine Verriegelungsfunktion auf, welche die beweglichen Arme der Klammer in einer geöffneten Position sichert, bis die Klammer für den Gebrauch positioniert ist, wobei die Verriegelungsfunktion der Arme der Klammer gelöst werden kann und die Klammer dann durch eine Vorspannung um den Knöchel des Patienten fest fixiert wird. Die Arme sind dabei zwischen einer offenen und einer geschlossenen Position bewegbar und werden mit Federn vorgespannt, um die geschlossene Position einzunehmen, die den Knöchel des Patienten während des Einsatzes greift. Die Arme weisen dafür einen gekerbten Endabschnitt auf, welcher sich in der Nähe des Drehpunktes des Armes befindet. Diese Kerbe arbeiten mit einer Verriegelung zusammen, um die Arme in einer geöffneten Position zu sichern. Die Verriegelung verfügt über eine außenliegende Druckplattenoberfläche, die manuell gedrückt werden kann, um die Verriegelung vom jeweiligen, beweglichen Arm zu lösen.

Ein Problem des Standes der Technik ist jedoch, dass die Fixierung am Knöchel des Patienten entweder lediglich kraftschlüssig erfolgt bzw. die Klammerarme kraftschlüssig vorgespannt und nicht formschlüssig fixiert werden (wodurch sich durch die dadurch entstehende Eigenelastizität der Klammerarme eine nur ungenügende Positionierbarkeit ergibt), oder dass zur Fixierung Schrauben in einen (gesunden) Knochen geschraubt werden müssen (was den Patienten zusätzlich belastet und auch das Infektionsrisiko erhöht). Dies führt im Falle der kraftschlüssigen Verbindung zu einer lösbaren Fixierung, die jedoch aufgrund der hohen Anforderungen an eine Maßhaltigkeit bzw. Genauigkeit an die eingangs beschriebene Ausrichtung der Ebene der Resektion nicht ausreichend ist, und führt, im Falle der Fixierung mittels Schrauben, zu einer weiteren, unnötigen Schädigung des Knochens, welche jedoch n unterbunden werden soll. Auch können durch die Klemmkraft elastisch vorgespannter Klammerarme Hämatome an den Körperstellen des Patienten entstehen. Des Weiteren werden durch die Fixierungsklammern nach dem Stand der Technik nicht alle anatomischen Größen von Patienten abgedeckt, da die Klemmkraft abhängig von der jeweiligen Anatomie des Patienten ist. Daher müssen verschiedene Varianten von Fixierungsklammern / Fixierungssysteme hergestellt und bereitgestellt werden.

### Zusammenfassung der Erfindung

Es ist die Aufgabe der Erfindung, die Nachteile aus dem Stand der Technik zu vermeiden oder wenigstens zu mindern und insbesondere ein Fixierungssystem sowie eine Ausrichtungsvorrichtung zur Verfügung zu stellen, das bzw. die eine einfache, sichere und schnelle Fixierung sowie ein einfaches und schnelles Lösen der Fixierung von einer Körperextremität / eines Körpergliedes, insbesondere um das Sprunggelenk bzw. um die Tibia des Patienten, ermöglichen, wobei das Fixierungssystem für unterschiedliche Anatomien von Körperextremitäten, insbesondere des Sprunggelenks, angepasst ist und für jede Anatomie eingesetzt werden kann und durch seine Funktionsweise und Konfiguration Hämatome vermeidet. Auch soll das Fixierungssystem und die Ausrichtungsvorrichtung mit nur einer Hand bedienbar sein.

Die Aufgabe der Erfindung wird hinsichtlich eines gattungsgemäßen Fixierungssystems erfindungsgemäß durch die Merkmale des Anspruchs 1 und hinsichtlich einer gattungsgemäßen Ausrichtungsvorrichtung erfindungsgemäß durch die Merkmale des nebengeordneten Anspruchs 10 gelöst.

Die Aufgabe der Erfindung wird bei einem gattungsgemäßen Fixierungssystem erfindungsgemäß dadurch gelöst, dass das Fixierungssystem einen Fixier-Magneten aufweist, der an einer Stirnseite oder an einem endständigen / stirnseitigen Abschnitt der Trägervorrichtung angeordnet / angebracht ist und zusammen mit der Trägervorrichtung einen Magnetträger bildet, sowie ein separat ausgebildetes, insbesondere flexibles, Magnetband aufweist, das bzw. dessen Vorderseite zu dem Fixier-Magnet anziehend ausgebildet ist, so dass der Fixier-Magnet an der Vorderseite des Magnetbands lösbar magnetisch haftet. Das Magnetband ist dafür angepasst, an Gliedmaßen / Körperextremitäten eines Patienten angebracht zu werden und weist insbesondere eine konvexe Krümmung auf um einen Durchmesser zu bilden. Gegenüber dem Stand der Technik weist das erfindungsgemäße Fixierungssystem also keine Klammerarme / Klemmfinger oder Klemmsysteme auf, bei denen Hämatome auftreten können, und keine Fixationsschrauben für eine Fixierung in einem Knochengewebe, sondern ein, vorzugsweise flexibles, Magnetband, das an der vorgesehenen Körperstelle angebracht und dort fixiert wird, und einen hierzu komplementären Fixier-Magneten, der an dem Magnetband lösbar magnetisch haftet. Eine Anbindung mittels Magnetkraft ist eine gänzlich neue Variante eines Fixierungssystems. Ein Magnetband kann dabei sowohl ein permanentmagnetisches Material als auch ein ferromagnetisches Material aufweisen. Ebenfalls kann der Fixier-Magnet ein permanentmagnetisches Material als auch ein ferromagnetisches Material aufweisen. Entscheidend ist nur, dass sich das Magnetband und der Fixier-Magnet magnetisch anziehen. Dies kann insbesondere dadurch geschehen, dass entweder das Magnetband oder der Fixier-Magnet ein permanentmagnetisches Material aufweisen.

Der Kern der vorliegenden Erfindung besteht demzufolge darin, statt eines Fixierungssystems mit im Wesentlichen nur einer einzigen / unteilbaren Baugruppe, das nach dem Stand der Technik mittels üblichen formschlüssigen Umgreifens des Knöchels mittels federelastisch vorgespannten Klammerarmen bzw. Klammerfingern einer an eine Ausrichtungsvorrichtung starr und fest angebundenen Fixierungsklammer realisiert wird, dem Anwender ein Fixierungssystem zur Verfügung zu stellen, das im Grunde genommen zumindest zweiteilig ausgeführt / mit zwei Komponenten bzw. Baugruppen ausgebildet ist, wobei die eine Komponente (das Magnetband bzw. das ferromagnetische Metallband) des Fixierungssystems formschlüssig, reibschlüssig und/oder stoffschlüssig an dem Knöchel des Patienten angeordnet und fixiert wird und die andere Komponente (der Magnetträger) an der Ausrichtungsvorrichtung befestigt wird. Die beiden Komponenten weisen dabei eine magnetische Wechselwirkung bzw. magnetische Anziehung auf, weshalb sie sich magnetisch mit einer magnetischen Maximalkraft anziehen. Diese Magnetkraft dient dazu, die beiden Komponenten des Fixierungssystems kraftschlüssig lösbar miteinander zu verbinden und folglich gegeneinander zu positionieren. So kann das Magnetband als die eine Komponente an dem Patienten unabhängig zu dem Magnetträger als andere Komponente befestigt, gelöst sowie neu positioniert werden und an dem Patienten verbleiben, wohingegen der Magnetträger magnetisch kraftschlüssig gelöst oder verbunden werden kann. Dies ermöglicht ein magnetisch bedingtes einfaches, leichtes und schnelles Anbringen/Fixieren und Lösen des Magnetträgers und damit der Ausrichtungsvorrichtung am (Sprunggelenk des) Patienten. Die Kombination aus einem Magnetband und einem Magnetträger als magnetischer Gegenpart ist gänzlich neu. Es werden Hämatome vermieden und das Magnetband passt sich anatomisch an den Patienten an. Das zur Verfügung gestellte Fixierungssystem kann mit einer Hand schnell und einfach bedient werden.

Vorteilhafte Ausführungsformen sind in den Unteransprüchen beansprucht und werden nachfolgend erläutert

In einer ersten bevorzugten Ausführungsform kann das Magnetband in Form eines Magnet-Schnappbandes gestaltet sein, dessen Längsachse sich in einem ersten Zustand (Offen-Zustand) durch eine geometrische Restriktion entgegen einer Vorspannung, bedingt durch eine symmetrisch zu der Längsachse zu einer Außenseite hin weisende Biegung, sich (im Wesentlichen) entlang einer Geraden erstreckt, und dessen Längsachse sich in einem zweiten Zustand (Schnapp-Zustand), nach Aufhebung der geometrischen Restriktion, die Vorspannung abbauend, spiralförmig oder wendelförmig um ein Körperglied herum windet und das Magnet-Schnappband dieses klemmend kraftschlüssig fixiert.

Vorzugsweise kann das Magnet-Schnappband entlang seiner Längsachse mehrere flexible magnetische Abschnitte aufweisen. Hierdurch wird ein magnetischer Bereich erweitert und eine genaue Ausrichtung des Magnet-Schnappbandes entfällt, da ja mehrere magnetische Abschnitte vorhanden sind.

Insbesondere kann das Schnappband entlang seiner Längsachse eine Länge von mindestens 300mm aufweisen, so dass es sich um ein Fußgelenk über 360° herumwinden kann. Dies gewährleistet eine ausreichende Klemmkraft bzw. neben der kraftschlüssigen auch eine formschlüssige Fixierung und bewirkt insbesondere, dass rundherum eine magnetisch anziehende Fläche um das Fußgelenk herum vorhanden ist.

In einer alternativen, zweiten bevorzugten Ausführungsform kann das Magnetband in Form einer, insbesondere flexiblen, Magnetfolie gestaltet sein, deren Rückseite einen Klebeabschnitt aufweist, um auf eine Körperstelle eines Körperglieds aufgeklebt zu werden. Der Klebeabschnitt ermöglicht also eine schnelle Fixierung auf einer Körperstelle und, wenn die Magnetfolie flexibel gestaltet ist, passt sich die Magnetfolie an die entsprechende Körperstelle anatomisch an. Damit kann mit nur einer einzigen Magnetfolie ein breites Spektrum an Anatomien abgedeckt werden. Die Magnet-Folie bietet dem Fixier-Magneten eine Fläche zum Andocken bzw. magnetischen Befestigen.

In einer weiteren, dritten alternativ bevorzugten Ausführungsform kann das Magnetband in Form eines Magnet-Armbandes bzw. Magnet-Fußbandes mit einem Verschluss ausgestaltet sein, welches an ein Glied umgreifend / umfassend angebracht und mittels des Verschlusses verschließbar und fixierbar ist. Der Verschluss kann dabei unterschiedliche Formen haben. Beispielsweise kann der Verschluss in Form eines Uhrenarmband-Verschlusses mit eine Dornschließe, welche in Öffnungen des anderen Uhrenarmbandes steht, ausgebildet sein. Auch kann der Verschluss als Klick-Schnellverschluss, oder als Clipverschluss ausgestaltet sein. Dies ermöglicht eine Fixierung des Magnet-Armbandes mit unterschiedlichen "Durchmessern" bzw. Umfängen und unterschiedlichen Verschlusskräften bzw. Fixierkräften entsprechend der Wahl der Enge des Armbandes.

Gemäß einem Aspekt der Erfindung kann die Trägervorrichtung eine Trägerbasis mit dem Anbindungsabschnitt und einen gegenüber der Trägerbasis, insbesondere mittels einer Führungsleiste, translatorisch in eine Verschieberichtung V verschiebbaren Schlitten mit dem Fixier-Magneten aufweisen. Insbesondere ist die Trägerbasis T-förmig ausgestaltet und der Schlitten bewegt sich an der Stirnseite der Trägerbasis.

Gemäß einem weiteren Aspekt der Erfindung kann die Trägervorrichtung eine Dreheinstellung/Drehvorrichtung/Dreheinstellvorrichtung aufweisen, mit der in der Verschieberichtung V die Position des Schlittens gegenüber der Trägerbasis einstellbar ist. Hierdurch kann mit einfachen Mitteln, insbesondere mittels einer Schnecke und eines zugehörigen Gewindes, sehr genau eine relative Position zwischen Trägerbasis und Schlitten eingestellt und auch beibehalten werden. Insbesondere weist die Dreheinstellung einen axialfest an der einen Komponente gehaltenen Gewindestift mit Außengewinde (Schnecke) auf, der in ein Innengewinde an der jeweils anderen Komponente eingreift.

Vorzugsweise kann die Trägervorrichtung eine senkrecht zu der Längsachse des Anbindungsabschnitts verlaufende konkave Fassung aufweisen, in die der Fixier-Magnet eingefasst ist, so dass zusätzlich zu der kraftschlüssigen Fixierung ein Formschluss zu den Seiten einer Tibia-Achse hin erfolgt. Durch die Fassung wird eine Fixierung weiter unterstützt und ein Freiheitsgrad quer zu der Tibia-Achse gesperrt.

In einer bevorzugten Ausführungsform kann der Fixier-Magnet von einer Kunststoffschicht ummantelt sein, so dass die Trägervorrichtung mit dem Fixier-Magneten gut sterilisierbar ist.

Vorzugsweise ist der Anbindungsabschnitt in Form eines rechteckförmigen Kragarms gestaltet, der entlang seiner Längsachse einen Durchgangsschlitz aufweist, um einen Einstellstab in Längsachsenrichtung (längs-)verschieblich aufzunehmen. Somit kann der Anbindungsabschnitt auch symmetrisch zu einem Einstellstab liegen.

Insbesondere weist der Anbindungsabschnitt an seinen Außenseiten Einkerbungen oder Riffelungen auf, um eine Position gegenüber einem Einstellstab mittels Formschluss festlegen zu können.

Die Aufgabe der Erfindung wird bei einer gattungsgemäßen Ausrichtungsvorrichtung für eine tibiale Resektionsführung zur Verwendung bei einer Vorbereitung eines Kniegelenks zur Implantation einer Prothese, mit einem Einstellstab, welcher an der Tibia angeordnet werden kann, und mit einer Führungsvorrichtung am proximalen Ende des Einstellstabes, um während einer Resektion der Tibia ein Werkzeug zu führen, erfindungsgemäß dadurch gelöst, dass ein am distalen Ende des Einstellstabes oder zum distalen Ende des Einstellstabes hin angeordnetes distales erfindungsgemäßes Fixierungssystem vorgesehen ist. Die Ausrichtungsvorrichtung lässt sich durch das erfindungsgemäße Fixierungssystem schnell und einfach an einem Schienbein bzw. gegenüber einer Tibia des Patienten ausrichten, anordnen und ebenso einfach wieder lösen, um die Ausrichtungsvorrichtung neu zu positionieren oder zu entfernen. Eine Handhabung wird deutlich vereinfacht und Hämatomen wird vorgebeugt.

### Kurzbeschreibung der Figuren

Die vorliegende Erfindung wird nachfolgend anhand bevorzugter Ausführungsformen unter Bezugnahme auf die begleitenden Figuren näher erläutert.
- Fig. 1: zeigt eine perspektivische Ansicht einer erfindungsgemäßen Ausrichtungsvorrichtung einer bevorzugten Ausführungsform,
- Fig. 2: zeigt eine perspektivische Ansicht eines erfindungsgemäßen Fixierungssystems einer ersten bevorzugten Ausführungsform,
- Fig. 3: zeigt eine perspektivische Ansicht des Fixierungssystems aus Fig. 2,
- Fig. 4: zeigt eine weitere perspektivische Ansicht des Fixierungssystems aus Fig. 2 und Fig. 3,
- Fig. 5: zeigt eine Draufsicht au ein Schnappband des Fixierungssystems der ersten bevorzugten Ausführungsform,
- Fig. 6: zeigt eine perspektivische Ansicht eines Fixierungssystems einer zweiten bevorzugten Ausführungsform mit Magnetfolie, und
- Fig. 7: zeigt eine perspektivische Ansicht eines Magnet Armbandes eines erfindungsgemäßen Fixierungssystems einer dritten bevorzugten Ausführungsform.

Die Figuren sind schematische Naturen sollen dem Verständnis der Erfindung dienen. Gleiche Elemente mit denselben Bezugszeichen versehen. Die Merkmale der verschiedenen Ausführungsformen können untereinander ausgetauscht werden.

### Beschreibung bevorzugter Ausführungsformen

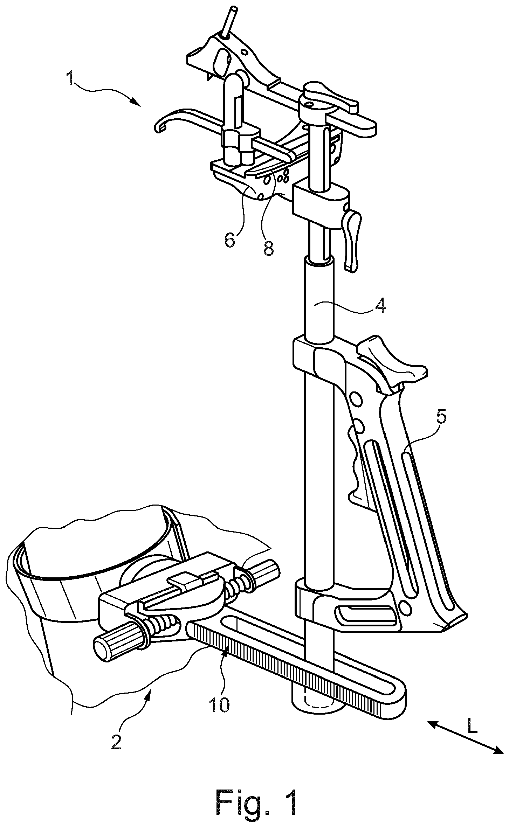

Figur 1 zeigt in einer perspektivischen Ansicht eine erfindungsgemäße Ausrichtungsvorrichtung 1 einer bevorzugten Ausführungsform für eine tibiale Resektionsführung zur Verwendung bei der Vorbereitung eines Kniegelenks zur Implantation einer Kniegelenksprothese mit einem erfindungsgemäßen Fixierungssystem 2 einer ersten bevorzugten Ausführungsform.

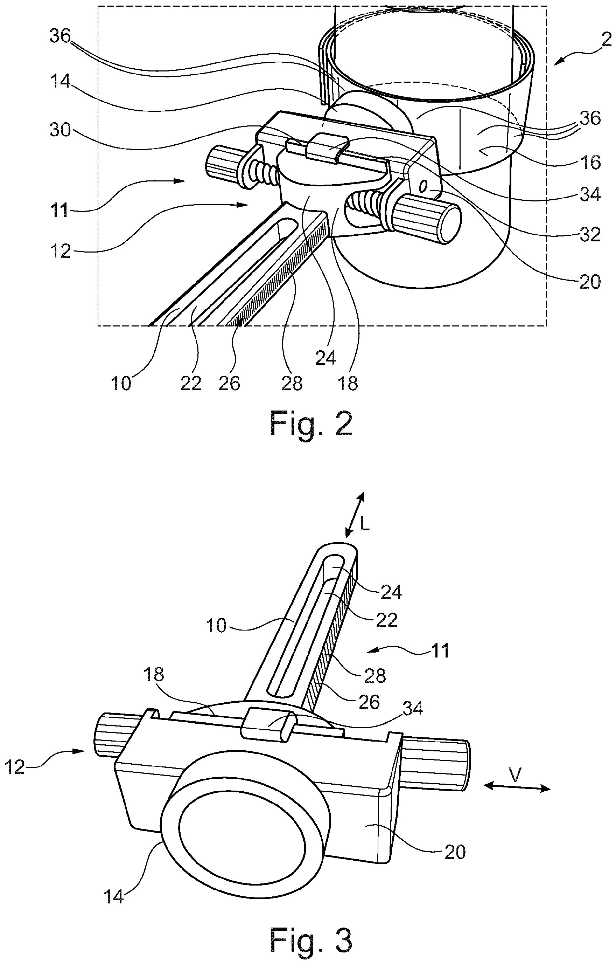

Die Ausrichtungsvorrichtung 1 weist einen längenverstellbaren Teleskopstab 4 mit einem daran starr montierten Handgriff 5 auf, sodass der Teleskopstab 4 gegenüber einer Tibia eines Patienten (nicht dargestellt) ausgerichtet werden kann. An einem Ende des Teleskopstabs 4, das bei Ausrichtung an dem Patienten zum Oberschenkel hin weist, ist eine (Werkzeug-) Führungsvorrichtung 6 in Form eines (Säge-)Blocks mit einem planen Durchgangsschlitz bzw. Durchgangsöffnung durch den Block als Schneidspalt bzw. Führungsspalt 8 befestigt, durch welchen ein Werkzeug hindurch gesteckt bzw. durchgeführt werden kann. Der (plane) Schneidspalt 8 legt dabei die Ebene der Resektion fest. An einem der Führungsvorrichtung 6 gegenüberliegendem Ende des Teleskopstabs 4, das bei Ausrichtung an dem Patienten zu dem Fuß hin zeigt, ist das erfindungsgemäße Fixierungssystem 2, welches nachstehend unter Bezugnahme der Figuren 2 bis 7 detailliert beschrieben wird, angelenkt bzw. befestigt. Das Fixierungssystem 2 dient der Festlegung eines Fixpunktes für die Ausrichtungsvorrichtung 1 und umgreift / umfasst dafür einen Knöchel bzw. ein Sprunggelenk des Patienten.

Das Fixierungssystem 2 weist zur Anbindung/Befestigung/Montage an den Teleskopstab 4 einen blockförmigen Kragarm / Kragträger 10 als Anbindungsabschnitt auf. Der Kragarm 10 weist entlang seiner Längsachse L eine Durchgangsöffnung in Form eines Durchgangsschlitzes 22 auf, wobei das dem Fuß hin zugewandte Ende des Teleskopstabs 4 in den Durchgangsschlitz 22 hinein- bzw. hindurchsteht und dabei bevorzugt die beiden durch den Durchgangsschlitz 22 gebildeten Spangen des Kragarms 10 elastisch auseinanderdrückt. Durch das geometrische Zusammenwirken kann das Fixierungssystem 2 translatorisch gegenüber dem Einstellstab 4 längsverschoben werden, um eine Position in der Sagittalebene bzw. einen Versatz zwischen dem Fixierungssystem 2 und der Führungsvorrichtung 8 einzustellen. Des Weiteren kann der Kragarm 10 mit einer (zusätzlichen) Vorspanneinrichtung ausgerüstet sein (am freien Ende des Kragarms 10 symbolisch dargestellt), über welche die Klemmkraft der beiden Spangen des Kragarms 10 auf den durch diese eingespannten Teleskopstab 4 weiter erhöht werden kann.

Wie im Übrigen aus der Fig. 1 zu entnehmen ist, können am Handgriff 5 eine Anzahl von Handhabungen (Betätigungstasten/Betätigungsräder) vorgesehen sein, mittels denen beispielsweise Funktionen des Teleskopstabs 4 aktuierbar sind.

Die Figuren 2 bis 5 zeigen das erfindungsgemäße Fixierungssystem 2 gemäß der ersten bevorzugten Ausführungsform. Das Fixierungssystem 2 weist dabei im Wesentlichen zwei Komponenten auf. Die eine Komponente bzw. Baugruppe ist ein Magnetträger 11 mit einer T-förmigen Trägervorrichtung 12 und mit einem stirnseitig angebrachten Fixier-Magneten 14 und die andere Komponente bzw. Baugruppe ist ein Magnetband in Form eines Schnappbands 16, dessen Vorderseite gegenüber dem Fixier-Magnet 14 magnetisch anziehend ausgebildet ist, insbesondere indem das Schnappband 16 mit einem magnetischen Material beschichtet ist oder in das Schnappband 16 magnetisches Material als Inlay bzw. Zwischenschicht eingebracht ist.

Zunächst wird der Magnetträger 11 mit der Trägervorrichtung 12 und dem Fixier-Magnet 14 unter Bezugnahme der Figuren 2 bis 4 detailliert beschrieben. Dieser Magnetträger 11 ist Bestandteil aller drei (verschiedenen) Ausführungsformen. Die Baugruppe der Trägervorrichtung 12 weist im Wesentlichen zwei Komponenten auf, nämlich zum einen eine T-förmige Trägerbasis 18 mit dem Kragarm 10 und zum anderen einen hierauf quer zu der Längsachse L des Kragarms 10 gleitend gelagerten (Träger-)Schlitten 20.

Die Trägerbasis 18 ist, wie bereits vorstehen erläutert, mittels des Kragarms 10 dafür angepasst, an dem Teleskopstab 4 der Ausrichtungsvorrichtung 1 verschieblich angelenkt zu werden. Für eine Einstellmöglichkeit einer Ausrichtung gegenüber der Ausrichtungsvorrichtung 1 weist die Trägerbasis 18 den blockförmigen bzw. stabförmigen Kragarm 10 auf, der das lange "T"-Stück der T-förmigen Trägerbasis 18 bildet und sich geradlinig von dem Schlitten 20 bzw. Fixier-Magneten 14 aus weg erstreckt. Der Durchgangsschlitz 22 weist abgerundete Endanschläge 24 auf, dieden gleichen Radius wie der Radius des Teleskopstabs 4 in diesem Bereich aufweisen. Damit kann der Teleskopstab 4 durch den Durchgangsschlitz 22 hindurchstehen und von einem Ende bis zu dem anderen Ende der Durchgangsschlitzes 22 längsverschieblich geführt werden. Die Außenseitenflächen 26 des Kragarms 10 weisen über ihre gesamte Abmessung entlang der Längsachse L Riffelungen bzw. Einkerbungen 28 auf, die senkrecht zu der Längsachse L des Kragarms 10 verlaufen. Mittels einer Positionsklammer (nicht dargestelllt) in Zusammenwirken mit den Einkerbungen 28 kann der Teleskopstab 4 dann an seiner vorgesehenen Stelle formschlüssig fixiert werden.

Die Trägerbasis 18 weist ferner an seiner Stirnseite, orthogonal zu der Längsachse L, zwei Führungsvorsprünge bzw. Führungsleisten 30 in eine Verschieberichtung V auf, wobei die Führungsleisten 30 versetzt zueinander auf zwei gegenüberliegenden Seiten der Trägerbasis einstückig mit dieser ausgebildet sind. Die beiden Führungsleisten 30 dienen der Führung des Schlittens 20, der auf einer planen Gleitoberfläche 32 der Trägerbasis 18 stirnseitig auf dieser translatorisch gleiten kann. Zwei sich gegenüberliegende Führungshaken / Umgreifungen 34 des Schlittens 20 umgreifen jeweils die Führungsleiste 30, so dass der Schlitten 20 axialverschieblich auf der Trägerbasis 18 geführt ist und nicht abfällt.

Auf der den Umgreifungen 34 gegenüberliegenden Seite bzw. der Stirnseite des Schlittens 20 ist der runde Fixier-Magnet 14 starr montiert. Alternativ ist natürlich auch vorstellbar, dass der Fixier-Magnet 14 in den Schlitten 20 bzw. in eine Einbuchtung in den Schlitten 20 eingelassen ist, sodass dieser bündig mit dem Schlitten 20 abschließt. Insbesondere kann der Fixier-Magnet 14 auch von einer Schutzschicht in Form einer Kunststoffschicht ummantelt sein, so dass eine Sterilisierbarkeit deutlich verbessert wird.

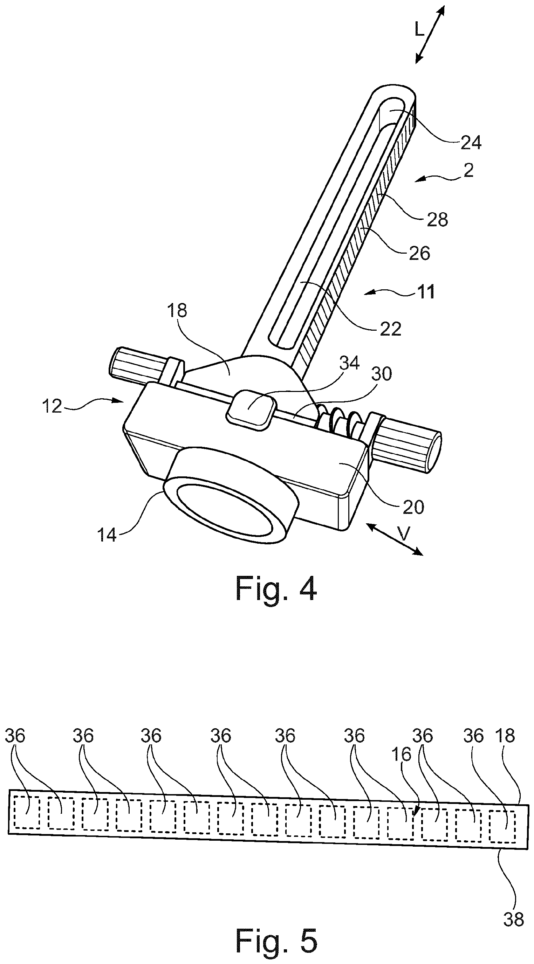

Das Schnappband 16 ist in Fig. 5 in einem ersten Zustand gezeigt, in dem seine Längsachse entlang einer Geraden verläuft. Für eine Magnetisierung weist das Schnappband 16 in dieser Ausführungsform entlang seiner gesamten Längsachse verteilt magnetische Abschnitte in Form von biegbaren Magnetblättchen 36 auf, die zwischen zwei Schichten des Schnappbands 16 eingelegt bzw. eingefasst sind. Das Schnappband 16 liegt plan bzw. langgestreckt dar. Dieser erste (Offen-) Zustand des Schnappbandes 16 wird durch eine geometrische Restriktion bewirkt, welche das Schnappband 16 entgegen seiner Vorspannung in seiner langgestreckten Stellung hält. Konkret weist das Schnappband 16 über seine Längsachse im Querschnitt gesehen eine leichte Biegung zu seinen Außenseiten 38 hin auf. Im Querschnitt betrachtet ist das Schnappband 16 also nicht ganz plan, sondern weist eine leicht konkave bzw. rinnenförmige Form auf. Diese rinnenförmige Form hindert das Schnappband 16 an einem Einrollen. Das langgestreckte Schnappband 16 kann dann an einem Schienbein oder an einem Sprunggelenk des Patienten (nicht dargestellt) positioniert werden. Wird manuell die leichte Biegung des Schnappbandes 16 dann zurückgebogen bzw. zurückgenommen, und damit die geometrische Beschränkung zurückgenommen, so windet sich das Schnappband aufgrund seiner materialbedingten elastischen Vorspannung, welche sie versucht zurückzubauen, um das Sprunggelenk des Patienten und klemmt dieses kraftschlüssig ein. Ein solches Schnappband 16 weist in seiner unverspannten Eigenform eine spiralförmige oder wendelfömige Form auf, wie sie in Fig. 2 gezeigt ist.

Die Magnetblättchen 36 sind entlang der gesamten Längsachse des Schnappbandes 16 mit diskretem Abstand angeordnet. Diese unterstützen sogar magnetisch, zusätzlich zu der Schnappwirkung des Schnappbandes 16, einen klemmendenVerschluss des um das Sprunggelenk spiralförmig anliegenden Schnappbandes 16.

Das Schnappband 16 weist im Querschnitt gesehen mehrere Lagen bzw. Schichten auf. Eine oberste und unterste Schicht dient der Abgrenzung des Schnappbandes 16 gegenüber seiner Umgebung und ist aus Kunststoff hergestellt. Die plane unterste und oberste Oberfläche kann gut gereinigt und sterilisiert werden. Zwischen der obersten und untersten Schicht ist eine Schnappform, die entweder blechartig oder kunststoffartig ausgeführt sein kann, sowie radial außen (siehe Fig. 2) über dieser gelegen, eines der flexiblen Magnetblättchen 36 angeordnet. Die oberste und unterste Schicht ummantelt also die Schnappform und das Magnetblättchen vollständig und grenzt diese nach außen hin ab.

Insbesondere kann das Fixierungssystem 2 verschiedene Schnappbänder 16 unterschiedlicher Länge und, im spiralförmigen Zustand, unterschiedlicher Durchmesser haben, so dass dem Anwender ein Set zur Verfügung gestellt wird, bei dem er, je nach Anatomie der Patienten, das passende Schnappband 16 auswählen kann, so dass der Durchmesser in etwa dem Durchmesser es Schienbeins nahe dem Sprunggelenk entspricht.

Ein solches Schnappband 16 ist auch produktionstechnisch günstig herstellbar, da die Magnetblättchen 36 für unterschiedliche Längen von Schnappbändern von den Abmessungen stets gleich ausgebildet sein können. Lediglich die Anzahl der Magnetblättchen 36 variiert je nach Länge.

Die Trägervorrichtung 12 weist für eine Ausrichtung des Schlittens 20 gegenüber der Trägerbasis 18 eine Drehspindel 40 als Dreheinstellung auf. Ein drehbarer Gewindestift / eine drehbare Gewindespindel 42 mit Außengewinde 44 greift dabei in einen Adapterblock mit Innengewinde ein, der an der Trägerbasis 18 montiert ist. Der Gewindestift 42 ist steht durch ein Bohrloch eines Steg hindurch und ist durch diesen axialfest an dem Schlitten 20 befestigt, so dass eine Drehung des Gewindestifts 42, entsprechend der Steigung des Außengewindes 44, eine translatorische Verschiebung des Adapterblocks mit Innengewinde und damit des Schlittens 20 in die Verschieberichtung V bewirkt. Mit der Drehspindel 40 kann also eine relative Position und damit eine Ausrichtung des Schlittens 20 gegenüber der Trägerbasis 18 eingestellt werden. Damit kann das Fixierungssystem 2 der Ausrichtungsvorrichtung 1unter anderem eine Anpassung der Neigung der Führungsvorrichtung 6 und damit des Führungsspaltes 8 bzw. der Resektionsebene bewirken.

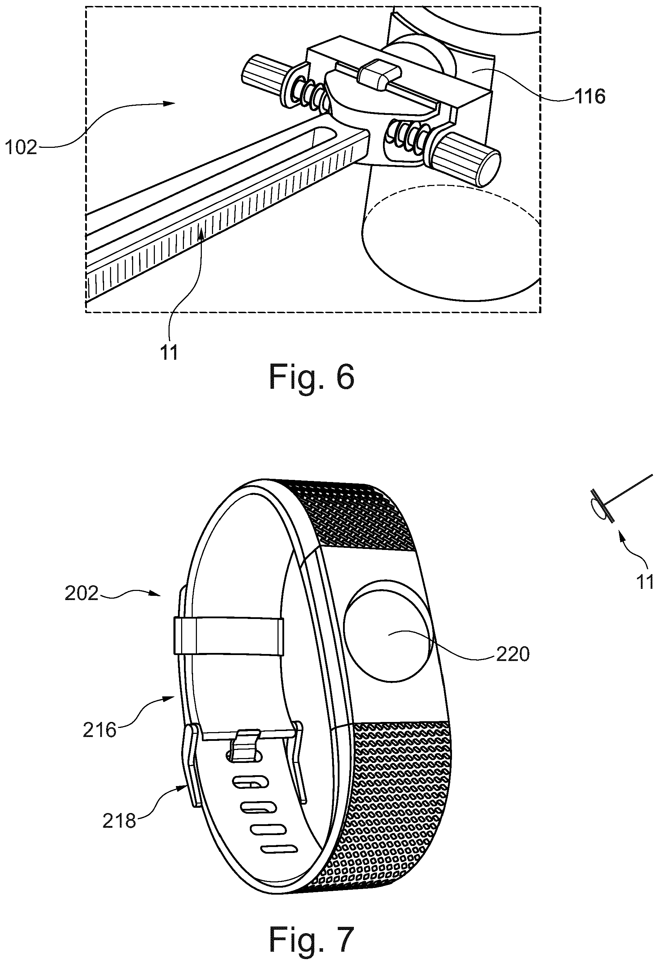

Figur 6 zeigt ein erfindungsgemäßes Fixierungssystem 102 gemäß einer zweiten bevorzugten Ausführungsform. Der Magnetträger 11 mit der Trägervorrichtung 12 und dem stirnseitigen Fixier-Magneten 14 ist wieder gleich zu der vorstehend beschriebenen ersten Ausführungsform ausgebildet, weswegen auf diese verwiesen wird. Im Unterschied zu der ersten Ausführungsform des Fixierungssystems 2 weist die zweite Ausführungsform des Fixierungssystems 102 nun statt eines Magnetbandes in Form eines Schnappbandes 16 (siehe Fig. 5), ein Magnetband in Form einer Magnetfolie 116 auf.

Die flexible und blattförmig gestaltete Magnetfolie 116 weist eine Schichtdicke von etwa 1 mm auf, ist auf der Vorderseite zu dem Fixier-Magnet 14 hin magnetisch anziehend und weist auf der Rückseite einen Klebeabschnitt in Form einer Klebeschicht auf. Die Klebeschicht weist für eine Handhabung eine die Klebeschicht schützende Abziehfolie auf. Hierdurch kann der Anwender die Magnetfolie 116 handhaben und vorpositionieren, ohne dass die Magnetfolie 116 ungewollt festklebt, und erst an der vorgesehenen rasierten Körperstelle kann die Abziehfolie entfernt und die flexible Magnetfolie 116 aufgebracht und klebend am Patienten fixiert werden.

Der Vorteil der Ausführungsform des Fixierungssystems 102 mit Magnetfolie 116 ist, dass die Magnetfolie 116 unabhängig von der Anatomie des Patienten an dessen Knöchel oder dessen unteren Schienbein angebracht werden kann. Die flexible Magnetfolie 116 passt sich adaptiv an die Oberfläche an. Nach der Resektion bzw. Operation wird die Magnetfolie vom Patienten, ähnlich eines Pflasters, wieder abgezogen und entfernt. Es entstehen keine Hämatome und die flexible Magnetfolie 116 deckt jegliche Anatomie eines Patienten ab. Auch ist die Magnetfolie 116 beschriftbar oder es können Markierungen aufgemalt werden.

Figur 7 zeigt ein erfindungsgemäßes Fixierungssystem 202 gemäß einer dritten bevorzugten Ausführungsform. Das Fixierungssystem 202 weist wieder den Magnetträger 11 auf, wobei nun, statt eines magnetischen Schnappbandes 16 oder einer Magnetfolie 116, ein Magnet-Armband 216 als Magnetband verwendet wird. Das Magnet-Armband 216 weist, ähnlich einem Uhrenarmband, einen in der Umfangsrichtung und damit den Durchmesser verstellbaren Armband-Verschluss 218 auf, um, an einem Sprunggelenk angelegt, je nach Anatomie das Magnet-Armband 216 fest zu zurren zu fixieren. Das Magnet-Armband 216 weist dem Verschluss 218 diametral gegenüberliegend einen magnetischen Armbandabschnitt 220 auf. Dieser ist wieder derart gestaltet, dass, angelegt um das Sprunggelenk des Patienten, die radial nach außen zeigende Vorderseite des magnetischen Armbandabschnitts 220 anziehend zu dem Fixier-Magneten 14 ist.

Der Vorteil des Magnet-Armbandes 216 ist, dass der magnetische Armbandabschnitt 220 plan gestaltet ist, um mit dem ebenso planen Fixier-Magneten 14 stärker und besser zu halten. Auch kann ein vorbestimmter magnetischer Bereich im Vorfeld festgelegt werden. In dieser Ausführungsform ist eine zu der geometrischen runden Form des Fixier-Magneten 14 komplementäre geometrische Einbuchtung 222 in dem magnetischen Armbandabschnitt 220 ausgebildet. Damit kann der Fixier-Magnet 14 durch den Anwender exakt in die Einbuchtung 222 positioniert werden, und ein sicherer Halt wird gewährleistet. Natürlich ist es auch möglich, das Magnet-Armband 216 ohne Einbuchtung 222 mit nur einer planen Oberfläche auszuführen. Dies vermeidet reinigungsintensive Hinterschnitte und gibt dem Anwender eine erweiterte Einstellmöglichkeit, da dieser den Fixier-Magneten 14 dann an unterschiedlichen Positionen in dem gesamten Bereich des magnetischen Armbandabschnittes 220 positionieren kann.

Der in dem magnetischen Abschnitt 220 eingelegte Magnet ist plan ausgeführt. Die geometrische Form des flexiblen Manget-Armbandes 216 hingegen passt sich der Anatomie des Sprunggelenks bzw. des Knöchels an, den es umschließt. Insbesondere kann als Werkstoff für das Magnet-Armband ein flexibler Kunststoff oder eine flexible Gummierung Anwendung finden.

Es sind natürlich weitere Verschlussarten des Maget-Armbandes 216 vorstellbar. Beispielsweise kann ein Rastmechanismus, ein weiterer magnetischer Verschlussmechanismus, ein Clip-Schnellverschluss, wie man ihn beispielsweise bei Rucksäcken vorfindet, oder ähnlich eines Gürtels eine (Verschluss-)Schnalle oder ein Clip-Verschluss verwendet werden.

### Bezugszeichenliste

- 1: Ausrichtungsvorrichtung
- 2; 102; 202: Fixierungssystem
- 4: Teleskopstab
- 5: Handgriff
- 6: Führungsvorrichtung
- 8: Führungsspalt
- 10: Kragarm
- 11: Magnetträger
- 12: Trägervorrichtung
- 14: Fixier-Magnet
- 16: Schnappband
- 18: Trägerbasis
- 20: Schlitten
- 22: Durchgangsschlitz
- 24: Abgerundete Seite
- 26: Lange Seitenfläche
- 28: Einkerbung
- 30: Führungsleiste
- 32: Gleitoberfläche
- 34: Umgreifung
- 36: Magnetischer Abschnitt
- 38: Außenseite
- 40: Dreheinstellung
- 42: Gewindestift
- 44: Außengewinde
- 116: Magnetfolie
- 216: Magnetarmband
- 218: Armband-Verschluss
- 220: Magnetischer Armbandabschnitt
- 222: Einbuchtung

- V: Verschieberichtung
- L: Längsachse

## Patentansprüche

1. Fixierungssystem (2; 202; 212) für eine Ausrichtungsvorrichtung (1) einer tibialen Resektionsführung zur fixierenden Anbindung an Gliedmaße, mit einer Trägervorrichtung (12), die einen Anbindungsabschnitt (10) zur Anbindung an einen Einstellstab aufweist, **gekennzeichnet durch**
einen Fixier-Magneten (14), der an einer Stirnseite der Trägervorrichtung (12) angeordnet ist, und
ein Magnetband (16; 116; 216), das zu dem Fixier-Magneten (14) anziehend ausgebildet ist und dafür angepasst ist, an Gliedmaßen eines Patienten angebracht zu werden.

2. Fixierungssystem (2) nach Anspruch 1, **dadurch gekennzeichnet, dass** das Magnetband in Form eines Magnet-Schnappbandes (16) gestaltet ist, dessen Längsachse sich in einem ersten Zustand durch eine geometrische Restriktion entgegen einer Vorspannung sich entlang einer Geraden erstreckt, und dessen Längsachse sich in einem zweiten Zustand, nach Aufhebung der geometrischen Restriktion, die Vorspannung abbauend, spiralförmig oder wendelförmig um ein Glied herum windet und das Magnet-Schnappband (16) klemmend zu fixieren.

3. Fixiersystem (102) nach Anspruch 2, **dadurch gekennzeichnet, dass** das Schnappband entlang seiner Längsachse eine Länge von mindestens 300mm aufweist.

4. Fixierungssystem (102) nach Anspruch 1, **dadurch gekennzeichnet, dass** das Magnetband in Form einer Magnetfolie (116) gestaltet ist, dessen Rückseite einen Klebeabschnitt aufweist, um auf eine Körperstelle aufgeklebt zu werden.

5. Fixierungssystem (202) nach Anspruch 1, **dadurch gekennzeichnet, dass** das Magnetband in Form eines Magnet-Armbandes (216) mit einem Verschluss (218) ausgestaltet ist, welches an ein Gliedmaß umgreifend angebracht, mittels des Verschlusses verschließbar und fixierbar ist.

6. Fixierungssystem (2; 102; 202) nach einem der Ansprüche 1 bis 5, **dadurch gekennzeichnet, dass** die Trägervorrichtung (12) eine Trägerbasis (18) mit dem Anbindungsabschnitt (10) und einen gegenüber der Trägerbasis (18), insbesondere mittels einer Führungsleiste (30), translatorisch in eine Verschieberichtung (V) gleitend verschiebbaren Schlitten (20) mit dem Fixier-Magneten (14) aufweist.

7. Fixierungssystem (2; 102; 202) nach Anspruch 6, **dadurch gekennzeichnet, dass** die Trägervorrichtung (12) eine Dreheinstellung (40) aufweist, mit der in der Verschieberichtung (V) die Position des Schlittens (20) gegenüber der Trägerbasis (18) einstellbar ist.

8. Fixierungssystem (2; 102; 202) nach einem der vorangegangenen Ansprüche, **dadurch gekennzeichnet, dass** die Trägervorrichtung (12) eine senkrecht zu der Längsachse (L) des Anbindungsabschnitts (10) verlaufende konkave Fassung aufweist, in die der Fixier-Magnet (14) eingefasst ist, so dass zusätzlich zu der kraftschlüssigen Fixierung ein Formschluss zu den Seiten einer Tibia-Achse erfolgt.

9. Fixierungssystem (2; 102; 202) nach einem der vorangegangenen Ansprüche, **dadurch gekennzeichnet, dass** der Anbindungsabschnitt (10) in Form eines Kragarms gestaltet ist, der entlang seiner Längsachse (L) einen Durchgangsschlitz (22) aufweist, um einen Einstellstab in Richtung der Längsachse (L) verschieblich aufzunehmen.

10. Ausrichtungsvorrichtung (1) für eine tibiale Resektionsführung mit einem Einstellstab (4), welcher an der Tibia angeordnet werden kann, und mit einer Führungsvorrichtung (6) am proximalen Ende des Einstellstabes (4), um während einer Resektion der Tibia ein Werkzeug zu führen, **gekennzeichnet durch**
ein am distalen Ende des Einstellstabes (4) oder zum distalen Ende des Einstellstabes (4) hin angeordnetes Fixierungssystem (2; 102; 202) nach einem der Ansprüche 1 bis 9.

## Claims

1. A fixing system (2; 202; 212) for an aligning device (1) of a tibial resection guide for fixating attachment to limbs, comprising a carrier device (12) having an attachment portion (10) for attaching to an adjustment rod, **characterized by**
a fixing magnet (14) arranged on a face side of the carrier device (12), and
a magnetic band (16; 116; 216) adapted to be attracted to the fixing magnet (14) and adapted to be attached to limbs of a patient.

2. The fixing system (2) according to claim 1, **characterized in that** the magnetic band is designed in the form of a magnetic slap wrap (16), the longitudinal axis of which in a first state extends along a straight line by a geometric restriction against a pretension, and the longitudinal axis of which in a second state, after the geometric restriction has been removed, winds around a limb in a spiral or helical manner, relieving the pretension and clampingly fixing the magnetic slap wrap (16).

3. The fixing system (102) according to claim 2, **characterized in that** the slap wrap has a length of at least 300 mm along its longitudinal axis.

4. The fixing system (102) according to claim 1, **characterized in that** the magnetic band is configured in the form of a magnetic foil (116), the back of which has an adhesive portion to be adhered to a body part.

5. The fixing system (202) according to claim 1, **characterized in that** the magnetic band is in the form of a magnetic wristband (216) with a closure (218), which is attached in a wrapping manner to a limb and can be closed and fixed by means of the closure.

6. The fixing system (2; 102; 202) according to one of claims 1 to 5, **characterized in that** the carrier device (12) has a carrier base (18) with the attachment portion (10) and a slider (20) with the fixing magnet (14), wherein the slider can be displaced translationally in a sliding direction (V) relative to the carrier base (18), in particular via a guide bar (30).

7. The fixing system (2; 102; 202) according to claim 6, **characterized in that** the carrier device (12) has a rotational adjustment mechanism (40) with which the position of the slider (20) relative to the carrier base (18) can be adjusted in the sliding direction (V).

8. The fixing system (2; 102; 202) according to one of the preceding claims, **characterized in that** the carrier device (12) has a concave holder perpendicular to the longitudinal axis (L) of the attachment portion (10), in which the fixing magnet (14) is enclosed, so that in addition to the force-fitting fixation, a form fit to the sides of a tibial axis is achieved.

9. The fixing system (2; 102; 202) according to one of the preceding claims, **characterized in that** the attachment portion (10) is in the form of a cantilever having a passage slit (22) along its longitudinal axis (L) for displaceably accommodating an adjustment rod in the direction of the longitudinal axis (L).

10. An aligning device (1) for a tibial resection guide having an adjustment rod (4) which can be arranged at the tibia and having a guiding device (6) at the proximal end of the adjustment rod (4) for guiding a tool during a resection of the tibia, **characterized by**
a fixing system (2; 102; 202) according to one of the claims 1 to 9 arranged at the distal end of the adjustment rod (4) or towards the distal end of the adjustment rod (4).

## Revendications

1. Système de fixation (2 ; 202 ; 212) pour un dispositif d'orientation (1) d'un guidage de résection tibial pour un raccordement fixant sur un membre, avec un dispositif de support (12) qui présente une section de raccordement (10) pour un raccordement sur une tige de réglage, **caractérisé par**
un aimant de fixation (14) qui est agencé sur un côté frontal du dispositif de support (12), et
une bande magnétique (16 ; 116 ; 216) qui est conçue pour attirer vers l'aimant de fixation (14) et adaptée pour être amenée contre des membres d'un patient.

2. Système de fixation (2) selon la revendication 1, **caractérisé en ce que** la bande magnétique est réalisée sous la forme d'une bande d'encliquetage-aimant (16) dont l'axe longitudinal, dans un premier état, s'étend le long d'une ligne droite par une restriction géométrique à l'encontre d'une précontrainte, et dont l'axe longitudinal, dans un second état, s'enroule en spirale ou en hélice autour d'un membre après levée de la restriction géométrique, éliminant la précontrainte, pour fixer par blocage la bande d'encliquetageaimant (16).

3. Système de fixation (102) selon la revendication 2, **caractérisé en ce que** la bande d'encliquetage présente le long de son axe longitudinal une longueur d'au moins 300 mm.

4. Système de fixation (102) selon la revendication 1, **caractérisé en ce que** la bande magnétique est réalisée sous la forme d'un film magnétique (116) dont le verso présente une section adhésive pour être collée sur un emplacement de corps.

5. Système de fixation (202) selon la revendication 1, **caractérisé en ce que** la bande magnétique est réalisée sous la forme d'un bracelet-aimant (216) avec un fermoir (218), lequel est amené contre un membre en l'enserrant, peut être fermé et fixé au moyen du fermoir.

6. Système de fixation (2 ; 102 ; 202) selon l'une des revendications 1 à 5, **caractérisé en ce que** le dispositif de support (12) présente une base de support (18) avec la section de raccordement (10) et une glissière (20), avec l'aimant de fixation (14), qui peut être déplacée par rapport à la base de support (18) par coulissement de façon translatoire dans une direction de déplacement (V), en particulier au moyen d'une barre de guidage (30).

7. Système de fixation (2 ; 102 ; 202) selon la revendication 6, **caractérisé en ce que** le dispositif de support (12) présente un réglage en rotation (40) avec lequel la position de la glissière (20) par rapport à la base de support (18) peut être réglée dans la direction de déplacement (V).

8. Système de fixation (2 ; 102 ; 202) selon l'une des revendications précédentes, **caractérisé en ce que** le dispositif de support (12) présente un cadre concave qui se déroule transversalement à l'axe longitudinal (L) de la section de raccordement (10), dans lequel cadre l'aimant de fixation (14) est encadré de sorte qu'en plus de la fixation par liaison de force se produise une liaison de forme sur les côtés d'un axe de tibia.

9. Système de fixation (2 ; 102 ; 202) selon l'une des revendications précédentes, **caractérisé en ce que** la section de raccordement (10) est réalisée sous la forme d'un bras en porte-à-faux qui présente une fente traversante (22) le long de son axe longitudinal (L) pour recevoir de façon déplaçable une tige de réglage dans la direction de l'axe longitudinal (L).

10. Dispositif d'orientation (1) pour un guidage de résection tibial avec une tige de réglage (4), laquelle peut être agencée au niveau du tibia, et avec un dispositif de guidage (6) à l'extrémité proximale de la tige de réglage (4) pour guider un outil pendant une résection du tibia, **caractérisé par**
un système de fixation (2 ; 102 ; 202) agencé à l'extrémité distale de la tige de réglage (4) ou en allant vers l'extrémité distale de la tige de réglage (4) selon l'une des revendications 1 à 9.
